Europäisches Patentamt

European Patent Office

Office européen des brevets

(11) Publication number: **0 167 345**
A1

# EUROPEAN PATENT APPLICATION

(21) Application number: **85304539.1**

(22) Date of filing: **26.06.85**

(51) Int. Cl.⁴: **F 16 B 2/00**

(30) Priority: **06.07.84 GB 8417260**

(43) Date of publication of application:
**08.01.86 Bulletin 86/2**

(84) Designated Contracting States:
**AT BE CH DE FR GB IT LI LU NL SE**

(71) Applicant: **MICRA Ltd.**
**54 Alma Street**
**Luton Bedfordshire LU1 2PL(GB)**

(72) Inventor: **Hoskin, William John**
**5 Long Buftlers**
**Harpenden Hertfordshire AL5 1JF(GB)**

(74) Representative: **Enskat, Michael Antony Frank et al,**
**Saunders & Dolleymore 2 Norfolk Road**
**Rickmansworth Hertfordshire WD3 1JH(GB)**

(54) Clamping arrangements.

(57) A snake assembly includes a plurality of elements (6) threaded on to a tensioning cable (12).

The elements (6) can be inclined relative to one another to allow the snake to adopt any desired curvature. When a tensioning device (20) is operated, the elements (6) are urged together so that they lock the snake in the curvature pattern adopted.

The contacting surfaces of the elements (6) are coated with smooth globules of tungsten or tungsten carbide. This provides the surface with an undulating random wave like topography. The effect is to provide a strong clamping action for a relatively low tensioning force in any relative position.

FIG.1

CLAMPING ARRANGEMENTS

The present invention relates to clamping arrangements, in particular for enabling the adjustable clamping of components.

Supports for surgical instruments and tools such as lighting units and microscopes is effected by a variety of multi-arm or multi-element devices. Such devices include a string of arms or elements with a holder at one end for supporting the tool or instrument and allow great versatility in the positioning of the holder. Once the holder has been positioned as required, adjoining elements or arms are clamped together under pressure to ensure that the holder remains in the required position.

The disadvantage of such arrangements is that considerable pressure must be applied to ensure that relative movement between adjacent arms or elements is prevented. Operators may have difficulty in applying such high pressures and the application of high pressures may induce harmful strains in the components themselves.

British Patent Specification No. 1,156,772 discloses a clamping arrangement in which the mating surfaces of two relatively slidable plates to be clamped together are provided with regularly arranged relatively large mating projections and depressions. With this arrangement, the two members can only be clamped together in a limited number of selected relative positions and so is unsatisfactory for use in situations where the two members are required to be clamped together in any relative positions. Furthermore, with regularly arranged projections and depressions, the two members must be rotated relatively to one another in alignment along a selected locus if a good clamping action is to be achieved.

It is an object of the invention to provide an improved clamping action between two members in whatever relative position the clamping surfaces of the members are urged together.

According to the present invention there is provided a clamping arrangement comprising two relatively slidable elements arranged to be urged together to lock the two elements against relative sliding movement, the contacting surfaces of each element having a topography consisting of randomly positioned smooth surfaced waves, the majority of waves falling into one of two different size ranges, the ratio of the two size ranges being greater than 70:1, whereby to allow relative sliding of the elements when the pressure urging the members together is relaxed.

According to the present invention there is also provided a clamping arrangement comprising two contacting members movable relative to one another, the contacting surface of each member having a randomly undulating but substantially smooth surface profile.

According to the present invention there is further provided a snake clamping assembly comprising a plurality of elements threaded onto a tensionable filament, each element having a spherical head at one axial end and a corresponding part-spherical recess at its opposite axial end, the head of each element being arranged to engage the recess of its adjacent element, the contacting surfaces of said elements having a randomly undulating but substantially smooth profile, and tensioning means for tensioning the threaded elements together along the filament, whereby to lock the elements in the relative attitudes in which they lie at that time.

According to the present invention there is still further provided a multi-arm support in which adjacent arms are secured together by a clamping arrangement, each clamping arrangement comprising at least two members urged into contact with one another and wherein the contacting surfaces of each said member comprises a randomly undulating but substantially smooth surface.

Clamping arrangements embodying the invention will now be described, by way of example, with reference to the accompanying diagrammatic drawings, in which:

Figure 1 is a perspective view of a 'snake' clamping assembly;

Figure 2 is a fragmentary section through the 'snake' clamping assembly of Figure 1;

Figure 3 is a perspective view of a multi-arm support;

Figure 4 is a section through one clamping arrangement of the support of Figure 3;

Figure 5 is a perspective view of one of the clamping surfaces; and

Figure 6 is a plan view of one of the clamping surfaces.

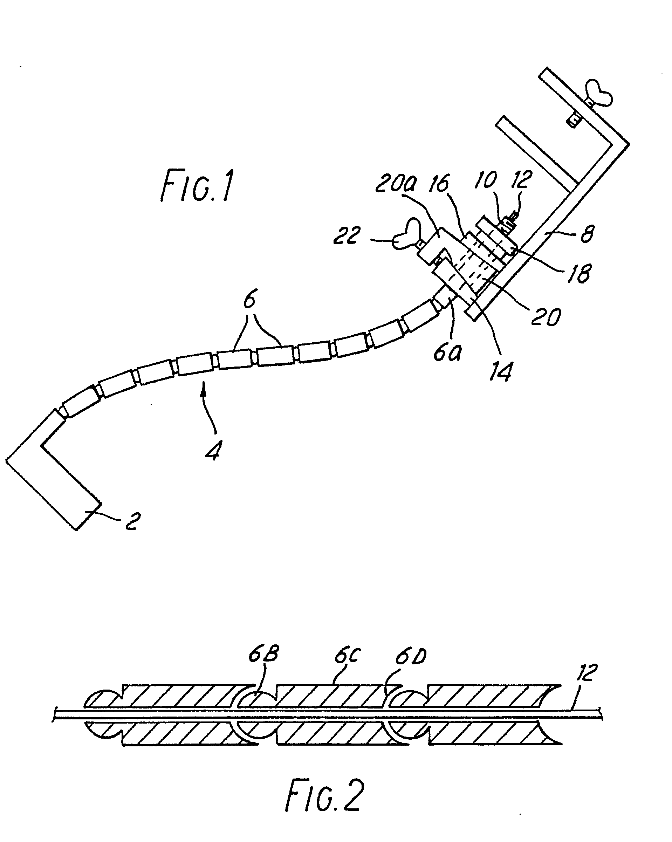

The 'snake' clamping assembly shown in Figure 1 comprises a holder 2 supported by a chain 4 of elements 6 from a support member 8. The holder 2 is linked to a screw-threaded rod 10 by a multi-stranded wire cable 12, and the elements 6, each of which has a central bore, are threaded onto the cable 12 (see Figure 2).

The end element 6a of the chain 4 is arranged to abut a stop member 14 rigid with the support member 8.

The rod 10 slidably carries an abutment member 18 and is in screw-threaded engagement with a knurled, internally screw-threaded, fastener 18. The facing surfaces of the stop member 14 and the abutment member 18 are mutually inclined and are engaged by a wedge-shaped cam member 20. The cam member 20 has a cantilevered portion 20a which extends along the outer surface of the stop member 14. The cantilevered portion 20a is traversed by, and in screw-threaded engagement with, a winged bolt 22 which has its free end in abutment with the outer surface of the stop member 14.

In operation, the winged bolt 22 is loosened to allow the wedge-shaped cam member 20 to slide in a sense in which the two members 14 and 16 can move towards one another. This reduces the tension in the cable 12 and so allows the individual elements to move relative to one another. In this state the holder 2 can be positioned as required. Once the desired position for the holder 2 is reached, the winged bolt 22 is tightened. When being tightened, the bolt 22 bears against the member 14 to cause the cam member 20 to slide between the members 14 and 16 and so urge them apart by cam action. As the member 16 is displaced away from the member 14, it engages the fastener 18 which in turn engages the rod 10 and so displaces the rod 10. The cable 12, which in turn is secured to the rod 10, is, thus tensioned to draw all the elements 6 together and in particular the end element 6a into tight abutment with the member 14. The elements 6 are thus clamped together to hold the holder 2 in the desired position and attitude.

The elements 6 are more clearly shown in Figure 2. Each element 6 has an elongate cylindrical body 6C carrying a spherical head 6B at one axial end and is provided with a part-spherical recess 6D at its other axial end.

The elements 6 are placed end to end so that the head 6B of one element matingly engages the part-spherical recess 6D in the adjacent element.

When the cable 12 is slack, the adjacent elements 6 can be displaced into relatively inclined positions. When the cable 12 is tensioned, the mating surfaces of adjacent elements provide a relatively large area of contact between elements 6 and so the frictional force inhibiting relative movement is relatively large.

While the frictional force between two elements can be increased by roughening, the surfaces, the sharp edges produced by such roughening makes relative movement between the elements difficult to effect and in any case such sharp edges will soon be worn smooth again with use.

In the case of the contacting surfaces of the elements 6, however, special treatment of these surfaces allows relative movement between the elements to be easily effected and produces a high clamping action under low tension conditions in the cable.

The mating contacting surfaces of the elements 6 (which are preferably of steel) are treated with a tungsten carbide electrode. The treatment involves the creation of a series of explosive sparks between the element and the tungsten carbide electrode. Because of the ionisation of the gas in the gap between the electrode and the element, minute particles of tungsten and tungsten carbide are carried across the gap and impinge upon the surface of the member 6 to be coated. This surface becomes locally molten at the time and so the particle becomes embedded in the surface.

The adhesion of particles deposited in this random way is particularly good and the deposited particles take the form of smooth curved flattish undulations with no rough edges.

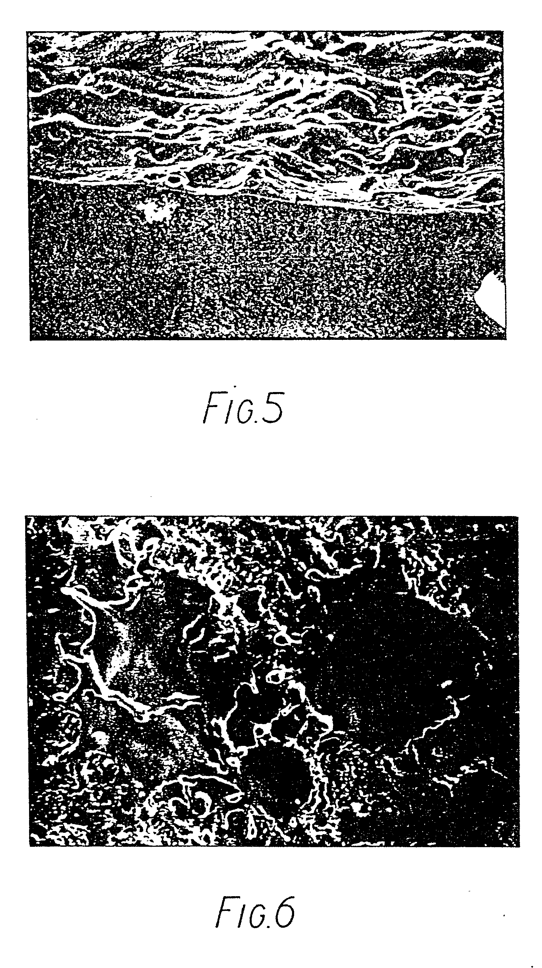

The configuration of the surface is more clearly illustrated in Figures 5 and 6.

The scale of the surface in Figure 5 is approximately 30 μm (0.03 mm) to the centimetre, while the scale of the surface of Figure 6 is approximately 15 μm (0.015 mm) to the centimetre.

As can best be seen in Figure 5, the topography consists of a plurality of randomly positioned different sized craters with a scattering of smaller sized globules.

From the perspective view shown in Figure 6, the surface appears akin to that of a choppy sea in motion frozen at a selected instant in time. As can be seen, the surface comprises a mixture of large and small waves, the large waves corresponding to the craters and the small waves corresponding to the globules. It will be apparent that the surfaces of both the large and small waves are generally smooth.

The large waves have an average pitch (diameter) which can vary in the range of from 50 to 150 μm (0.05 to 0.15 mm). The small waves have an average pitch (diameter) which can vary in the range of from 0.3 to 0.7 μm (0.0003 to 0.0007 mm). The ratio of the size of the large waves to small waves can vary over the range of from 500:1 to 70:1. The peak height of the waves (crest to trough) can be up to 8 μm (0.008 mm).

The formation of these topographic features results from local melting by the molten droplets impacting the surface and spreading laterally to produce the smooth areas. Breakup of the molten material around the periphery gives rise to the formation of small globules. The smooth areas are seen to partially cover a microscopically much rougher underlying surface structure and in some cases to have been deposited on top of other previously formed smooth areas. The microscopically rougher surface consists of small craters and locally melted and resolidified material and has a generally rounded topography.

The smooth nature of the surfaces enables two similar contacting surfaces to slide easily relative to one another in the absence of a load or pressure. When the surfaces are forced together under pressure, the peaks of the waves in one surface lock into the troughs of the waves in the other surface (and vice versa) in a random fashion and this provides a very rigid clamping action to inhibit relative sliding between the surfaces.

With the co-acting surfaces of the elements 6 coated in this manner, relative movement between them is easily effected when the cable is only under light tension and a good and effective clamping action is effected by producing only a moderate increase in the tension of the cable.

This clamping action is more effective because as indicated hereinbefore the flattish undulations on one of the contacting surfaces of one element fall into the hollows between the flattish undulations on the other element and so if relative movement between the contacting surfaces is to take place, not only must frictional forces be overcome but also the forces required in stretching the cable to allow the undulations of one element to ride over those of the other element.

Advantageously, the average height of the undulations is arranged to be in excess of 0.005 mm and the average surface area of each undulation is arranged to be less than 0.3 $mm^2$.

It will be appreciated that similar surfaces could be produced by liquid spray coating with other metals and also refractories such as alumina or zirconia. Liquid spray coating processes involve using materials of high melting point which are then liquified in or at the exit of a spray gun. Such coatings all have smooth globules and rely for adhesion on the fact that they are just molten when they reach the surface onto which they cling.

In yet another modification, the elements 6 could each be made of allumina and the coating surfaces sandblasted to produce a surface structure having the required characteristics.

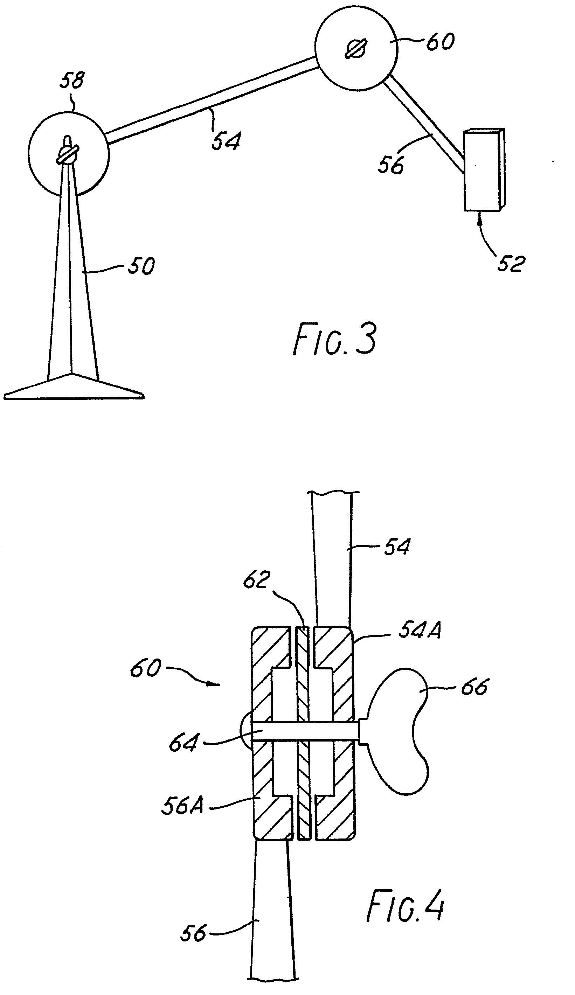

The multi-arm support shown in Figure 3 includes an upstanding support member 50 supporting a holder 52 through two linked arms 54 and 56. One clamping assembly 60 couples the arms 54 and 56 while another clamping assembly 58 couples the arm 54 to the upstanding support 50.

The two clamping assemblies 58 and 60 are similar and so only one is shown in more detail in Figure 4.

As shown, the arms 54 and 56 terminate in respective cup-shaped end portions 54A and 56A and are so orientated that the rims of the end portions face one another. A disc 62 is located between the facing rims. A bolt 64 passes centrally through the two cup-shaped end members 54A and 56A and a wing nut 56 screw-threadedly engages the nut 64. By turning the wing nut 66 the clamping assembly can be tightened or loosened.

The mating surfaces of the cup-shaped end portions 54 and 56 and the disc 62 all have surface profiles such as are described in connection with the mating surfaces of the elements 6 of the 'snake' clamping assembly of Figure 2. The materials of the surfaces can also be the same.

In another modification of the snake, the individual links, instead of defining balls and sockets to provide the contacting surfaces, can take the form of a series of cylindrical members in which each alternate member is positioned at right angles to each intervening member. Thus, each intervening member is axially threaded and each alternate member diagonally threaded to accommodate the cable 12.

CLAIMS

1.          A clamping arrangement comprising two relatively slidable elements arranged to be urged together to lock the two elements against relative sliding movement, the contacting surfaces of each element having a topography consisting of randomly positioned smooth surfaced waves, the majority of waves falling into one of two different size ranges, the ratio of the two size ranges being greater than 70:1, whereby to allow relative sliding of the elements when the pressure urging the members together is relaxed.

2.          An arrangement according to Claim 1, wherein the topography of each said surface is generated by causing globules of molten material to impinge upon and adhere to said elements.

3.          An arrangement according to Claim 2, wherein said globules are produced by spark erosion treatment of said elements with a tungsten carbide electrode.

4.          An arrangement according to any preceding Claim, wherein the average pitch of the waves in the larger of said size ranges lies in the range of from 50 to 150 μm.

5.          An arrangement according to any preceding Claim, wherein the average pitch of the waves in the smaller of said size ranges lies in the range of from 0.3 to 0.7 μm.

6.          An arrangement according to any preceding Claim, wherein the average crest to trough dimension of the waves exceeds 0.005 mm.

7.          A clamping arrangement according to any preceding Claim, comprising a plurality of said elements threaded on a filament to define a flexible snake, the drawing of said filament urging said elements together to hold the snake in the attitude adopted by the elements at the time of said drawing action.

8.        A clamping arrangement comprising two contacting members movable relative to one another, the contacting surface of each member having a randomly undulating but substantially smooth surface profile.

9.        An arrangement according to Claim 8, wherein each said contacting surface comprises a random distribution of substantially smooth flat undulations of different sizes adhering to its contacting member.

10.        An arrangement according to Claim 9, wherein each undulation and the local area of contact with a said member are each rendered molten to effect adhesion between the undulation and the member.

11.        An arrangement according to Claim 9 or to Claim 10, wherein the dimension of each undulation in a direction normal to the contacting surface is in excess of 0.005 mm.

12.        An arrangement according to any one of Claims 8 to 11, wherein the average surface area of each undulation is arranged to be less than 0.30 mm.

13.        An arrangement according to any one of Claims 8 to 12, wherein each undulation is of a material selected from the group consisting of tungsten, tungsten carbide, allumina and zirconia.

14.        A snake clamping assembly comprising a plurality of elements threaded onto a tensionable filament each element having a spherical head at one axial end and a corresponding part-spherical recess at its opposite axial end, the head of each element being arranged to engage the recess of its adjacent element, the contacting surfaces of said elements having a randomly undulating but substantially smooth profile, and tensioning means for tensioning the threaded elements together along the filament, whereby to lock the elements in the relative attitudes in which they lie at that time.

15.        An assembly according to Claim 14, wherein the contacting surfaces of said elements are formed by globules of tungsten or tungsten carbide.

16.        A multi-arm support in which adjacent arms are secured together by a clamping arrangement, each clamping arrangement comprising at least two members urged into contact with one another and wherein the contacting surfaces of each said member comprises a randomly undulating but substantially smooth surface.

17.        A multi-arm support according to Claim 16, wherein the clamping arrangement has a third member arranged to lie between said two first mentioned members.

18.        A multi-arm support according to Claim 16 or to Claim 17, wherein said contacting surfaces of said elements are formed by globules of tungsten or tungsten carbide.

19.        An arrangement or assembly according to Claim 8, Claim 14 or to Claim 16 wherein the effective part of the surface has smooth waves or undulations having a pitch in the range of 0.3 to 0.7 $\mu$m.

FIG.1

FIG.2

_Fig.3_

_Fig.4_

Fig.5

Fig.6

0167345

**European Patent Office**

## EUROPEAN SEARCH REPORT

Application number

| | | | |
|---|---|---|---|
| **DOCUMENTS CONSIDERED TO BE RELEVANT** | | | EP 85304539.1 |

| Category | Citation of document with indication. where appropriate, of relevant passages | Relevant to claim | CLASSIFICATION OF THE APPLICATION (Int Cl 4) |
|---|---|---|---|
| A | DE - A - 1 750 252 (POLAR HYDRAU-LICS LTD.)<br>* Page 1-6; fig. * | 1,7,8,14 | F 16 B 2/00 |
| A | DE - B - 2 514 496 (OLYMPUS OPTICAL CO., LTD.)<br>* Totality * | 1,7,8,14 | |
| D,A | GB - A - 1 156 772 (A.E. GAUTIER)<br>* Totality * | 1,8 | |
| A | GB - A - 1 462 735 (MICHELIN)<br>* Claims; fig. * | 1,8 | |
| A | GB - A - 1 436 703 (F. FLETCHER)<br>* Page 2, lines 35-121; fig. 1-3,5 * | 1,8 | **TECHNICAL FIELDS SEARCHED (Int Cl 4)** |
| A | EP - A1 - 0 094 520 (J.W.O. HÖISETH)<br>* Claim 1; fig. 1 * | 1,8 | F 16 B<br>A 61 B |
| A | DD - A - 85 883 (E. GOMILLE et al.)<br>* Page 4, line 15 - page 7 * | 1,8 | |
| A | CH - A5 - 640 313 (G. GENTILE)<br>* Totality * | 1,8 | |

The present search report has been drawn up for all claims

| Place of search | Date of completion of the search | Examiner |
|---|---|---|
| VIENNA | 13-08-1985 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82